(19)

Europäisches
Patentamt
European
Patent Office
Office européen
des brevets

(11)  **EP 4 556 062 A1**

(12)  **EUROPEAN PATENT APPLICATION**
published in accordance with Art. 153(4) EPC

(43) Date of publication:
**21.05.2025 Bulletin 2025/21**

(21) Application number: **23839079.3**

(22) Date of filing: **17.07.2023**

(51) International Patent Classification (IPC):
**A61N 5/06** (2006.01)      **A61F 9/00** (2006.01)

(52) Cooperative Patent Classification (CPC):
**Y02B 20/40**

(86) International application number:
**PCT/CN2023/107768**

(87) International publication number:
**WO 2024/012593 (18.01.2024 Gazette 2024/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **15.07.2022  CN 202210832712**

(71) Applicant: **Shanghai Airdoc Medical Technology
Co., Ltd.
Shanghai 200233 (CN)**

(72) Inventors:
• **HE, Chao
Shanghai 200233 (CN)**

• **CHANG, Xiangang
Shanghai 200233 (CN)**
• **LU, Peng
Shanghai 200233 (CN)**
• **LI, Da
Shanghai 200233 (CN)**
• **REN, Wenbin
Shanghai 200233 (CN)**
• **JIANG, Xin
Shanghai 200233 (CN)**
• **LI, Chun
Shanghai 200233 (CN)**

(74) Representative: **Boult Wade Tennant LLP
Salisbury Square House
8 Salisbury Square
London EC4Y 8AP (GB)**

(54)  **RED-LIGHT IRRADIATION CONTROL METHOD FOR MYOPIA PHYSIOTHERAPY, AND
RELATED PRODUCT THEREOF**

(57)  Disclosed is a red-light irradiation control method for myopia physiotherapy, including: acquiring an ocular surface image of a user (S102); analyzing the ocular surface image to determine state information of a pupil (S104); and controlling, based on the state information of the pupil and a red-light irradiation in-eye light power of the user, red-light irradiation on a fundus of the user (S106). With the red-light control solution of the present disclosure, safe, effective and personalized fundus red-light irradiation may be provided for each user according to the specific eye features (for example, a pupil state) and red-light irradiation in-eye light power of the user, so that the efficiency of myopia physiotherapy may be obviously improved.

Fig. 1

# Description

## CROSS-REFERENCE TO RELATED APPLICATION

This application claims priority to Chinese patent application No. 202210832712.4 titled "RED-LIGHT IRRADIATION CONTROL METHOD FOR MYOPIA PHYSIOTHERAPY, AND RELATED PRODUCT THEREOF" filed on July 15, 2022.

## TECHNICAL FIELD

The present disclosure generally relates to the optical field. More specifically, the present disclosure relates to a red-light irradiation control method, device, apparatus and computer-readable storage medium for myopia physiotherapy.

## BACKGROUND OF THE INVENTION

Currently, the myopia problem of teenagers is becoming more severe due to overuse of devices with electronic display screens. To prevent and slow down development of myopia, a solution of irradiating the fundus with low-intensity red light has been proposed. In terms of specific operation, a low-intensity red-light apparatus for irradiating the fundus may be used to irradiate with low-energy red laser on the macular region, so that beneficial sunlight rays can be accurately simulated. With such red-light irradiation, the blood circulation of the fundus can be improved, and dopamine secretion from retinal pigment epithelia can be promoted, so that the thinned choroid can be restored to normal. In addition, with the continuous irradiation of red light, the sclera can be supplied with sufficient oxygen, whereby the strength of the sclera can be enhanced. Finally, the irradiation of red light can help to inhibit abnormal growth of the eye axis, so that effective prevention, control and correction of myopia can be implemented.

Although the red-light irradiation as described above is effective for the prevention, control and correction of myopia, unified red-light irradiation operation often results in an actual red-light irradiation in-eye light power of a user failing to reach the expected or ideal value since the eye features (for example, different pupil states) vary with users. Therefore, the effect of myopia physiotherapy is limited, and even potential risks and injuries may occur. In view of this, how to effectively control the red-light irradiation to provide effective red-light irradiation for eyeballs has become a problem to be solved now.

## SUMMARY OF THE INVENTION

In view of the above-mentioned technical problem, the present disclosure provides a solution for controlling the red light irradiating the fundus. The control solution of the present disclosure can implement effective control and regulation of the irradiation power during red-light irradiation, thereby notably improving the effectiveness of red-light irradiation and myopia physiotherapy.

To this end, in a first aspect, the present disclosure provides a red-light irradiation control method for myopia physiotherapy, comprising: acquiring an ocular surface image of a user; analyzing the ocular surface image to determine state information of a pupil; and controlling, based on the state information of the pupil and a red-light irradiation in-eye light power of the user, red-light irradiation on a fundus of the user.

In one embodiment, acquiring the ocular surface image of the user includes acquiring the ocular surface image of the user by at least one positioning camera before and/or during red-light irradiation on the fundus of the user.

In one embodiment, analyzing the ocular surface image to determine the state information of the pupil includes analyzing the ocular surface image by a neural network model to determine the state information of the pupil.

In one embodiment, the state information of the pupil includes a pupil size.

In one embodiment, the state information of the pupil further includes a distance of the pupil relative to a red-light irradiation assembly.

In one embodiment, the state information of the pupil further includes a position and/or direction of the pupil relative to the red-light irradiation assembly.

In one embodiment, the method further comprises: moving the red-light irradiation assembly in at least one moving direction in response to a shift in the position and/or direction of the pupil relative to the red-light irradiation assembly, to provide vertical red-light irradiation on the fundus.

In one embodiment, controlling, based on the state information of the pupil and the red-light irradiation in-eye light power of the user, red-light irradiation on the fundus of the user includes: determining a light source power of the red-light irradiation assembly based on the pupil size and the red-light irradiation in-eye light power of the user; and controlling the red-light irradiation on the fundus of the user based on the light source power of the red-light irradiation assembly.

In one embodiment, the light source of the red-light irradiation assembly includes a non-homogenized light beam, and determining the light source power of the red-light irradiation assembly based on the pupil size and the red-light irradiation in-eye light power of the user includes: determining the light source power based on the pupil size, the red-light irradiation in-eye light power of the user, and a power-annulus radius correspondence relationship of the non-homogenized light beam.

In one embodiment, the light source of the red-light irradiation assembly includes a homogenized light beam, and determining the light source power of the red-light irradiation assembly based on the pupil size and the red-light irradiation in-eye light power of the user includes: determining the light source power based on the pupil size, the red-light irradiation in-eye light power of the user, and a power-annulus radius correspondence relationship of the homogenized light beam.

In one embodiment, controlling, based on the state information of the pupil and the red-light irradiation in-eye light power of the user, red-light irradiation on the fundus of the user further includes: determining the light source power of the red-light irradiation assembly based on the pupil size, the distance of the pupil relative to the red-light irradiation assembly, and the red-light irradiation in-eye light power of the user.

In one embodiment, the light source power of the red-light irradiation assembly ranges from 0.1mw to 1.7mw.

In one embodiment, a spectrum of the light source of the red-light irradiation assembly is narrowband red-light or infrared light, with a center wavelength within the range of 630nm to 850nm, and a width (full width at half maximum) less than 20nm.

In a second aspect, the present disclosure provides a red-light irradiation control device for myopia physiotherapy, comprising: a processor; and a memory having program instructions for controlling red-light irradiation on a fundus stored thereon which, when executed by a processor, cause the method according to the aforementioned first aspect and various embodiments thereof to be implemented.

In a third aspect, the present disclosure provides an apparatus for myopia physiotherapy, comprising: a positioning camera configured to capture an ocular surface of a user to generate an ocular surface image; a red-light irradiation assembly configured to irradiate red-light on a fundus of the user to implement myopia physiotherapy; and the device according to the second aspect, which is connected to the positioning camera and the red-light irradiation assembly, respectively, and configured to control the red-light irradiating the fundus.

In a fourth aspect, the present disclosure provides a computer-readable storage medium having program instructions for controlling red-light irradiation on a fundus stored thereon which, when executed by a processor, cause the method according to the aforementioned first aspect and various embodiments thereof to be implemented.

With the red-light irradiation control method of the present disclosure, safe, effective and personalized red-light irradiation on the fundus may be implemented for different users. Specifically, by, for example, acquiring an ocular surface image of a user in real time or near real time, and analyzing it, the solution of the present disclosure may determine the pupil size of the user, the distance, position, direction of the pupil relative to the red-light irradiation assembly, and other state information, and perform personalized control on the light source power of the red-light irradiation assembly according to the state information of the pupil. By controlling the light source power as described above, the solution of the present disclosure may ensure that the actual red-light irradiation in-eye light power of the user (*i.e.,* the actual red-light power irradiated on the fundus of the user) is consistent with the red-light irradiation in-eye light power of the user (also referred to as "standard red-light irradiation in-eye light power" or "expected red-light irradiation in-eye light power" of the user, which may be interchanged herein). Therefore, the solution of the present disclosure may implement effective control of red-light irradiation on the fundus, and avoid ineffective or inefficient irradiation on the fundus. In some embodiments, with the help of a neural network model in the field of artificial intelligence, the solution of the present disclosure may achieve accurate analysis of the ocular surface image, so that the condition of the pupil may be accurately determined to provide a good basis for subsequent power control or adjustment.

In some application scenarios, the solution of the present disclosure may be implemented before, during or after the red-light irradiation, and therefore, the solution of the present disclosure may be applied to different occasions to realize monitoring, which is flexible with time, of effectiveness of the red-light irradiation. When implemented in an apparatus, the solution of the present disclosure provides a myopia physiotherapy apparatus supporting controllable red-light irradiation, so that the effectiveness of red-light irradiation is ensured, and the treatment effect of the myopia physiotherapy is improved. In addition, with the red-light control solution of the present disclosure, safe, effective and personalized fundus red-light irradiation may be provided for each user according to each user's specific eye features (for example, a pupil state) and red-light irradiation in-eye light power of the user, so that the efficiency of myopia physiotherapy may be obviously improved.

## BRIEF DESCRIPTION OF THE DRAWINGS

The above and other objectives, features and advantages of the exemplary implementations of the present disclosure will become readily apparent from the following detailed description, which proceeds with reference to the accompanying drawings. In the accompanying drawings, several implementations of the present disclosure are illustrated by way of example but not limitation, and the same or the corresponding reference numerals indicate the same or the corresponding parts, in which:

FIG. 1 is a simplified flowchart of a red-light irradiation control method for myopia physiotherapy according to an embodiment of the present disclosure;
FIG. 2 is a detailed flowchart of a red-light irradiation control method according to an embodiment of the present disclosure;
FIG. 3 is a detailed flowchart of a red-light irradiation control method according to another embodiment of the present disclosure;

FIG. 4 is a principle block diagram of a red-light irradiation control device for myopia physiotherapy according to an embodiment of the present disclosure; and

FIG. 5 is a structural block diagram of an apparatus for myopia physiotherapy according to an embodiment of the present disclosure.

## DETAIL DESCRIPTION OF THE INVENTION

The technical solutions in the embodiments of the present disclosure will be clearly and completely described below with reference to the accompanying drawings in the embodiments of the present disclosure. Obviously, the described embodiments are only a part of the embodiments of the present disclosure, but not all of them. Based on the embodiments in the present disclosure, all other embodiments obtained by those skilled in the art without making creative efforts belong to the protection scope of the present disclosure.

As discussed above, to implement effective control of red-light irradiation on a fundus, in particular control of energy of the red-light irradiation on a fundus, a solution of the present disclosure is proposed to acquire state information of a pupil, such as pupil size information, by analyzing an ocular surface image of a user. Then, based on the state information and the acquired red-light irradiation in-eye light power of the user, a light source power of a red-light irradiation assembly for irradiation on the fundus may be effectively controlled, thereby significantly improving the effectiveness of the red-light irradiation on the fundus. A spectrum of the light source of the red-light irradiation assembly is narrow-band red-light or infrared light, with a center wavelength within the range of 630nm to 850nm, and a width (full width at half maximum) of the spectrum less than 20nm. The preferred wavelength is $650\pm10$nm. The light source power of the red-light irradiation assembly may range from 0.1mw to 1.7mw.

Specific implementations of the present disclosure will be described in detail below with reference to the accompanying drawings.

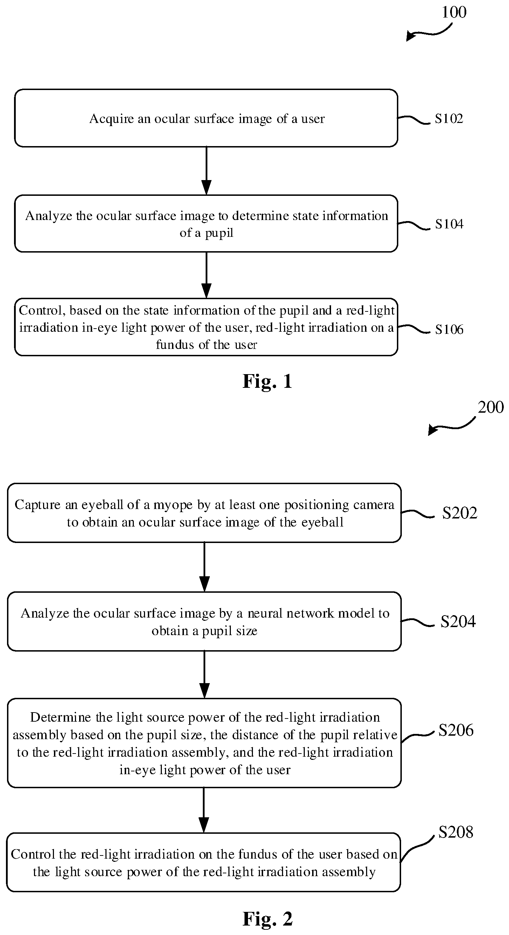

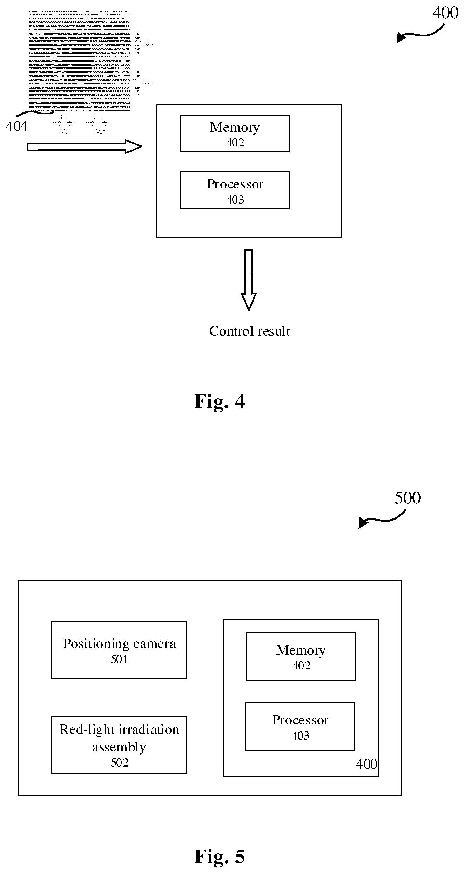

FIG. 1 is a simplified flowchart of a red-light irradiation control method 100 for myopia physiotherapy according to an embodiment of the present disclosure. It is to be understood that the red-light irradiation here may be performed by a red-light irradiation device (also called "assembly", which can be used interchangeably herein) for irradiating red light on the fundus. Specifically, the red-light irradiation device may firstly irradiate red light on an ocular surface of a myope, and then the red light irradiates the fundus through the pupil, thereby performing myopia physiotherapy on the eyeball. On this basis, the method 100 may be performed by a device (as shown in FIG. 4) that interacts with and controls the red-light irradiation device. Depending on different application scenarios, the control device may be an internal device integrated in the red-light irradiation device, or an external device connected to a control device or system of the red-light irradiation device.

As shown in FIG. 1, at step S102, an ocular surface image of a user is acquired. In one implementation scenario, the ocular surface image here may be captured by a positioning camera disposed at the red-light irradiation device, and transmitted to a processor in the device performing the method 100. As an example, two positioning cameras may be set up and positioned at a periphery of the red-light irradiation device or integrated inside the red-light irradiation device. The ocular surface of the eyeball may be captured, such as in real time or near real time way, by the positioning cameras to acquire an ocular surface image of the user.

The timing of the aforementioned acquiring the ocular surface image is not particularly limited in the present disclosure. The act of acquiring the ocular surface image may be performed before and/or during the red-light irradiation on the fundus.

At step S104, the ocular surface image is analyzed (for example, an analysis in the field of computer vision) to determine state information of a pupil. In one implementation scenario, the aforementioned state information of the pupil may include pupil size information. In another implementation scenario, the aforementioned state information of the pupil may further include a distance of the pupil relative to a red-light irradiation assembly. In yet another implementation scenario, the aforementioned state information of the pupil may further include a position and/or direction of the pupil relative to the red-light irradiation assembly.

When the aforementioned state information of the pupil includes the pupil size information, in some implementation scenarios, the aforementioned analysis may be, for example, computer analysis on the aforementioned ocular surface image captured by the two positioning cameras. As an example, the aforementioned computer analysis may include analyzing the ocular surface image by a neural network model in the field of artificial intelligence to determine the pupil size.

Finally, at step S106, based on the state information of the pupil and a red-light irradiation in-eye light power of the user, red-light irradiation on a fundus of the user is controlled. According to the principle of the solution of the present disclosure, the size or dimension of the pupil is related to the actual red-light irradiation in-eye light power of the user, so that the light source power of the red-light irradiation device may be adjusted based on the pupil size, to make the actual red-light irradiation in-eye light power of the user closer to the standard value, thereby promoting effective improvement of the eye myopia problem. Here, the red-light irradiation in-eye light power of the user may be obtained in different ways. As an example, an ophthalmologist may, after taking an examination of a user (e.g., a myope), determine an in-eye light power that is suitable for the red-light irradiation on the fundus of the user (i.e., the "standard red-light irradiation in-eye light power" or "expected red-light irradiation in-eye light power" mentioned above). As another example, a suitable red-light irradiation in-eye light power for the user may be determined by looking up a historic record of past red-light irradiation in-eye light powers of the user. In addition, various physiological indexes of the user related to myopia may be input into a computer system which generates a suitable red-light irradiation in-eye light power of the user. Therefore, it is to be understood that in-eye light power may be obtained in different ways depending on different application scenarios, which is not particularly limited in the solution of the present disclosure.

The control as described above may also be implemented in different ways. As an example, the control here may refer to adjusting the light source power based on the state information of the pupil *(e.g.,* the pupil size and the distance of the pupil relative to the red-light irradiation assembly) and the red-light irradiation in-eye light power. In some scenarios, the light source power may be adjusted by adjusting the distance between the pupil and the red-light irradiation device, to achieve the expected red-light irradiation in-eye light power of the user. Therefore, the power adjustment method of the present disclosure here is merely exemplary, and one skilled in the art, given the teachings of the present disclosure, may adjust the red-light irradiation in other ways to achieve effective irradiation on the fundus. For example, in some scenarios, in response to a shift in the position and/or direction of the pupil relative to the red-light irradiation assembly, a solution of the present disclosure further proposes to move the red-light irradiation assembly in at least one moving direction to align the pupil of the user with the light source of the red-light irradiation device, so that vertical red-light irradiation on the fundus, and therefore effective red-light irradiation on the fundus, are implemented.

Based on the state information and the selected red-light irradiation in-eye light power, the method of the present disclosure may determine the corresponding light source power. On this basis, before the next red-light irradiation is started, the light source power of the red-light source may be adjusted to an effective red-light irradiation light source power determined in a previous irradiation, to further improve the efficiency and effectiveness of the red-light irradiation. In addition, since different users may have different red-light irradiation in-eye light powers that suitable for each of them, the method of the present disclosure may enable personalized red-light irradiation. For example, irradiation of 0.3mW may be performed for user A, while irradiation of 0.35mW may be performed for user B.

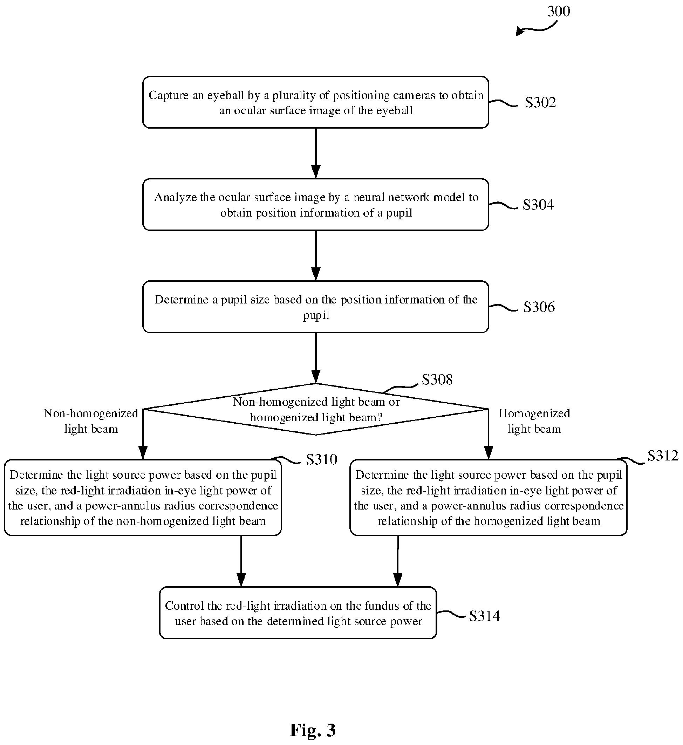

A specific processing scenario when an ocular surface image of an eyeball is acquired during red-light irradiation will be described below with reference to FIGs. 2 and 3, respectively. It is to be understood that the following specific operations are merely exemplary and not limiting, and that one skilled in the art, based on the disclosure and teachings herein, may recognize other specific ways to implement the present disclosure.

FIG. 2 is a detailed flowchart of a method 200 for controlling red-light irradiation on a fundus according to an embodiment of the present disclosure. It is to be understood that the following method 200 may be regarded as an implementation scenario of the method 100 shown in FIG. 1, and therefore, the description of the method 100 in conjunction with FIG. 1 in the above is also applicable to the description of the following method 200.

As shown in FIG. 2, at step S202, an eyeball of a myope is captured by at least one positioning camera to obtain an ocular surface image of the eyeball (shown as 404 in FIG. 4). Depending on different scenarios, the ocular surface image here may include a normal eye pupil image or an abnormally shaped pupil image. When the ocular surface is continuously imaged at a fixed frequency, the temporally spaced ocular surface images further reflect a certain degree of fluctuation in the pupil size, such as narrowing of the pupil after a period of irradiation. In some scenarios, the pupil in the ocular surface image may be obscured by upper and lower eyelids, and such situations are also within the scope of the present disclosure.

Next, at step S204, the ocular surface image is analyzed by a neural network model to obtain a pupil size. Depending on different applications, the neural network model herein may, for example, involve a convolutional neural network configured to predict a pupil position and/or a logistic regression model (which may be considered as a single layer neural network containing only one neuron) configured to predict a pupil position. The neural network may be trained repeatedly with ocular surface images containing the pupil, to finally obtain a neural network model which enables good inference operations *(i.e.,* positioning orientation and size of the pupil). Through the computation of such a neural network model, the pupil size in the ocular surface image may be determined. As known to those skilled in the art, an adult typically has a pupil diameter between 2mm and 4mm, and a pupil shape of a perfect circle. Generally, the pupil size not only varies with the intensity of light, but also is related to other factors such as age, refraction, and physiological conditions.

At step S206, the light source power of the red-light irradiation assembly is determined based on the pupil size, the distance of the pupil relative to the red-light irradiation assembly, and the red-light irradiation in-eye light power of the user. For example, the corresponding light source power may be determined by the following equation (1):

$$P_a = \frac{P_s(r)}{f1 \times f2} \quad (1)$$

where $P_s(r)$ represents the red-light irradiation in-eye light power, $P_a$ represents the light source power, $f1$ represents the distance of the pupil relative to the red-light irradiation assembly, and $f2$ represents the pupil

size.

Finally, at step S208, the red-light irradiation on the fundus of the user is controlled based on the light source power of the red-light irradiation assembly. It is to be understood that the above manner of determining the light source power from the red-light irradiation in-eye light power is merely exemplary, and those skilled in the art may contemplate other manners to adjust the red-light irradiation. For example, a mapping table of pupil sizes and light source powers may be directly established, so that when the pupil size is determined, the corresponding light source power may be directly found in the mapping table to perform the corresponding adjustment. For another example, when the red-light irradiation in-eye light power is obtained, the red-light irradiation device may be moved appropriately, directly according to the correspondence table of red-light irradiation in-eye light powers and distances between the red-light source and the ocular surface, so that the distance between the red-light source and the ocular surface may be adjusted to a suitable position. Based on such adjustment of the relative position, a region of the pupil irradiated by the red light is also changed accordingly, thereby improving the effectiveness of red-light irradiation on the fundus.

In one application scenario, the current in-eye light power or light source power may be finely tuned based on a previous adjustment and changes in the pupil size. For example, a pupil size in a previously captured ocular surface image may be compared with a pupil size obtained by analyzing a currently captured ocular surface image to identify a size difference between the two. Next, the current light source power may be adjusted (or finely tuned) based on this size difference, so that the adjusted red-light beam with the expected red-light irradiation in-eye light power may irradiate the fundus most effectively through the pupil.

FIG. 3 is a detailed flowchart of a method 300 for controlling red light irradiating a fundus according to another embodiment of the present disclosure. It is to be understood that the method 300 here includes more implementation details of the method 100 shown in FIG. 1, and therefore, the description of the method 100 in conjunction with FIG. 1 is also applicable to the method 300.

As shown in FIG. 3, at step S302, an eyeball may be captured by a plurality of (for example, two) positioning cameras, to obtain an ocular surface image of the eyeball during red-light irradiation. As discussed above, the positioning cameras here may be two positioning cameras provided by a red-light irradiation device, which may be mounted on two sides of a lens barrel of the red-light irradiation device. In addition to capturing the eyeball to obtain an ocular surface image of the eyeball, in some scenarios, the positioning cameras may be further used for precise position determination of the eyeball during red-light irradiation. For example, according to the principle of stereotactic positioning, a first positioning camera and a second positioning camera may acquire features of the ocular surface (*e.g.*, a pupil) from different positions and viewing angles, thereby determining a position of the eyeball or the pupil relative to the red-light irradiation device (*e.g.,* the lens barrel). Thereafter, the red-light irradiation assembly or the whole lens barrel may be moved based on the obtained position information, so that the red-light irradiation device may be aligned with the eye (such as the eyeball) of a myope, so that efficient red-light irradiation or imaging may be implemented.

Next, at step S304, the ocular surface image is analyzed by a neural network model to obtain position information of a pupil. As an example, the neural network model here may be implemented as an object detection device, where the object is a pupil in the context of the present disclosure. The object detection device may include a plurality of object detection modules, such as a first object detection module and a second object detection module. The first object detection module may process the ocular surface image to obtain preliminary position information of the pupil. From this preliminary position information, a pupil region image may be captured from the ocular surface image. Then, the second object detection module may be used to process the pupil region image to obtain final position information of the pupil. Referring to FIG. 4, the dotted rectangular box in the ocular surface image corresponds to a preliminary prediction rectangular box output from the first object detection module, and the solid rectangular box corresponds to a final prediction rectangular box output from the second object detection module. Further, the final position information may include final coordinates of a center point, a final width, and a final height of the pupil.

At step S306, a pupil size is determined based on the position information of the pupil. For example, the pupil size may be calculated from the obtained final position information as described above. Here, the pupil size may include a pupil size when the pupil is obscured by upper and lower eyelids, or a pupil size that is changed from a previous measurement. Next, at step S308, the type of red-light beam currently used by the red-light irradiation device may be determined, *i.e.,* non-homogenized light beam or homogenized light beam. It is to be understood that the light beam type determination here is an optional step. For example, for a red-light irradiation device having only one type of light beam, this determination step may be omitted.

When the irradiated light beam is non-homogenized *(i.e.,* non-homogenized light spots), the laser is a Gaussian beam. Therefore, at step S310, the light source power is determined based on the pupil size, the red-light irradiation in-eye light power of the user, and a power-annulus radius correspondence relationship of the non-homogenized light beam. By way of example only, a power-annulus radius correspondence relationship of Gaussian beam (which refers to a total light beam power within a coverage area of the annulus radius when the annulus radius is r, and when the annulus radius r represents the pupil radius, the Gaussian beam power represents the red-light irradiation in-eye light power) may be expressed by the following equation (2):

$$P_s(r) = \frac{2r}{R^2} exp^{-\frac{r^2}{R^2}} P_a \quad (2)$$

where $P_s(r)$ represents the red-light irradiation in-eye light power, R represents a light spot radius of the laser beam, r represents the pupil radius, and $P_a$ represents the light source power. Assuming in the red-light irradiation device, a light spot diameter of the light beam is 10mm, the pupil size is reduced to a diameter of 2mm, and the light source power Pa is 1mW, then the in-eye light power is 0.077mW. Further assuming that the red-light irradiation in-eye light power corresponding to the pupil size of a diameter of 2mm is expected to reach 0.1mW, then according to equation (2), the light source power of the red-light irradiation device should be adjusted to: 1*0.1/0.077=1.3mW.

Next, at step S314, the light source power of red-light irradiating the fundus may be adjusted based on the red-light irradiation in-eye light power.

When the irradiated light beam is homogenized, at step S312, the light source power is determined based on the pupil size, the red-light irradiation in-eye light power of the user, and a power-annulus radius correspondence relationship of the homogenized light beam. Specifically, in the case of homogenized light spots, the power that the annulus with a radius r corresponds to is

$$Pr = Sr/SR \times Pa \quad (3)$$

where Pr is the in-eye light power, Pa is a total light spot power, Sr represents a pupil area with a radius r, and SR represents an area of a light spot with a light spot radius R.

Still assuming that the homogenized light beam has a light spot diameter of 10mm and the light source power Pa is 1mW, then a pupil of 2mm corresponds to a red-light irradiation in-eye light power Pr of 0.04mW, which is shown as the following equation (4):

$$P_r = 1/5^2 \times 1 = 1mW \quad (4)$$

Assuming that the red-light irradiation in-eye light power Pr corresponding to the pupil diameter of 2mm is expected to reach 0.1mW, the light source power Pa of the red-light irradiation device may be adjusted to: Pa = 1*0.1/0.04=2.5mW.

Next, at step S314, the red-light irradiation on the fundus of the user is controlled based on the determined light source power.

Referring to the method 300 shown in FIG. 3, the present disclosure presents different controlling solutions of the red-light source power for different light beam types. It is to be understood that the method shown in FIG. 3 is merely exemplary and not limiting. Based on the disclosure and teachings of the present disclosure, one skilled in the art will appreciate that the red-light power of the present disclosure may also be adjusted in other manners, which also fall within the scope of the present disclosure.

FIG. 4 is a principle block diagram of a device 400 for controlling red-light irradiation on a fundus according to an embodiment of the present disclosure. It is to be understood that the device 400 may perform steps of the methods described in conjunction with FIGs. 1 to 3.

As shown in FIG. 4, the device 400 of the present disclosure includes a memory 402 and a processor 403, where the memory may store program instructions for controlling red-light irradiation for myopia physiotherapy. Additionally or alternatively, the memory 402 may further store codes for implementing an analysis algorithm on an ocular surface image. Depending on different implementation scenarios, the processor 403 here may be a general purpose processor or a special purpose processor (*e.g.*, an artificial intelligence processor). Further, when the program in the memory 402 is executed by the processor 403, the device will receive an ocular surface image 404, and perform steps of the methods described in conjunction with FIGs. 1 to 3 to finally output a control result for use in controlling a red-light source power of the red-light irradiation device.

FIG. 5 is a principle block diagram of an apparatus 500 for myopia physiotherapy according to an embodiment of the present disclosure. As shown in the figure, the apparatus 500 includes the device 400 which may implement procedures of the methods described in conjunction with FIGs. 1 to 3, where the device 400 for controlling red-light irradiation includes a memory 402 and a processor 403. As discussed above, the memory 403 stores program instructions for controlling red-light irradiation on a fundus, when program instructions are executed by a processor, steps of the methods described in conjunction with FIGs. 1 to 3 may be implemented.

Further, the apparatus 500 includes a positioning camera 501 and a red-light irradiation assembly 502, where the red-light irradiation assembly 502 may constitute what is referred to in the context of the present disclosure as a red-light irradiation device. As discussed above, in one implementation scenario, the positioning camera here may include two positioning cameras disposed on two sides of the red-light irradiation assembly, which may be operated to image an eyeball in real time or near real time to generate an ocular surface image of the eyeball. In another practical scenario, the positioning camera may be further operated to position the eyeball, particularly the pupil, so that a position of the apparatus 500 may be accurately adjusted according to the position of the eyeball to be aligned with the eyeball for efficient red-light irradiation. In addition, according to the images captured by the positioning camera, the processor 403 may further determine compliance of the user during the irradiation. The compliance may involve, for example, whether the user has squinted eyes, half-open eyes, closed eyes, or head tilt. When poor compliance of the user is detected, *i.e.,* when any of the above situations occurs, the apparatus 500 may notify the situation through voice broadcast or graphic display via an external interface, to prompt the user to correct the current compliance deviation, so that good red-light irradiation and myopia treatment effects may be obtained.

In one implementation scenario, the red-light irradiation assembly 502 includes a red-light source, which may be any of a variety of light source devices that include a laser head configured to emit low-intensity red light *(e.g.,* red light on the order of 650nm). The red light emitted from the light source device may pass through the pupil of the eyeball and irradiate on the fundus via, for example, a collimating lens arranged in the light path, so that the blood circulation of the fundus may be improved, and dopamine secretion from retinal pigment epithelia may be promoted. In addition, the thinned choroid may be restored to normal, while sufficient oxygen may be supplied to the sclera to enhance strength of the sclera. Finally, the effect of inhibiting abnormal growth of the eye axis may be achieved, thereby implementing effective prevention, control and correction of myopia.

Taking the control during red-light irradiation as an example, during operation of the apparatus 500, the device 400 for controlling red-light irradiation may be firstly activated in preparation for monitoring the red light to be used. Then, the red-light irradiation assembly of the red-light irradiation device may be turned on to irradiate the fundus with red light. During the irradiation, the positioning camera may be activated to capture an image of the ocular surface of the eyeball, thereby obtaining an ocular surface image.

Thereafter, the device 400 receives the ocular surface image from the positioning camera, and performs the flow steps shown in FIGs. 1 to 3 to implement control of the red-light irradiating the fundus. For example, after determining the pupil size by running a pupil positioning code, the processor 403 may determine the red-light source power corresponding to the current pupil size by computation or looking up a table or the like. Thereafter, the processor 403 may interact with the red-light irradiation assembly, through a bus for example, to instruct a control circuit in the red-light irradiation assembly to adjust a power of the red light emitted from the red-light source. Alternatively, the processor 403 may send instructions to a movement mechanism (not shown in drawings) in the apparatus 500, so that the movement mechanism may move the red-light source to an appropriate position to change a distance between the red-light source and the ocular surface. Therefore, the red-light source, emitted from the apparatus towards a window of the eye of the myope, will reach the fundus through the pupil with a more proper distribution, and the effectiveness of red-light irradiation is improved.

In some application scenarios, the apparatus 500 may further inform the user, via an external interface through voice broadcast or graphic display, of current size information of eyeball pupils of the user, and a magnitude of the red-light irradiation in-eye light power or incident light power for irradiating the fundus.

The apparatus for myopia physiotherapy of the present disclosure is described in detail above with reference to FIG. 5. It is to be understood, however, that the description here is merely exemplary and not limiting, and that based on the disclosure of the present disclosure, variations to the shown apparatus may be appreciated by those skilled in the art. For example, although the red-light irradiation device and the device for controlling the red-light irradiation device are depicted together, the two may be arranged separately in some application scenarios. For example, the red-light irradiation device may be arranged in a lens barrel with a window, while the device 400 may be arranged in a base of the apparatus, and the two may be connected through various connection interfaces (for example, wireless or wired interfaces, such as serial bus interfaces). In addition, in some application scenarios, the processing of ocular surface image analysis, pupil size determination, and control operations may be arranged on a remote server (*e.g.*, a cloud server), thereby reducing the size and computation cost of the apparatus 500.

In the above description of this application, the terms "fixed", "installed", "connected" or "coupled" or the like are to be construed broadly, unless otherwise expressly stated and defined. For example, the term "connected" may refer to components being fixedly connected, detachably connected, or forming an integral; or being mechanically connected or electrically connected; or being directly connected, indirectly connected via an intermedium, or two elements in internal communication or interaction. Therefore, those skilled in the art may understand the specific meanings of the above terms in the present disclosure according to the specific context, unless otherwise explicitly limited in the present application.

It will also be appreciated that any module, unit, component, server, computer, terminal or device exemplified herein that executes instructions may include or otherwise have access to computer-readable media such as storage media, computer storage media, or data storage devices (removable and/or non-removable), for example, magnetic disks, optical disks or tape. The computer storage medium may include volatile and non-volatile, removable and non-removable media implemented in any method or technology for storage of information, such as computer-readable instructions, data structures, program modules, or the like.

In addition, "first", "second" and other terms for indicating a number or sequence used herein are merely for the purpose of illustration and are not to be construed as indicating or implying a relative importance or implicitly indicating the number of the indicated technical features. Therefore, a feature defined by "first" or "second" may include at least one that feature either explicitly or implicitly. In the description of the present application, "a plurality of" means at least two, e.g., two, three or more or the like, unless explicitly defined otherwise.

Although various embodiments of the present disclosure have been shown and described herein, it will be obvious to those skilled in the art that such embodiments are provided by way of example only. Numerous modifications, changes, and substitutions may occur to those skilled in the art without departing from the spirit and scope of the present disclosure. It should be appreciated that various alternatives to the embodiments of the present disclosure described herein may be employed in practicing the present disclosure. It is intended that the following claims define the scope of the present disclosure and that the unit composition, equivalents, or alternatives falling within the scope of these claims are covered thereby.

## Claims

1. A red-light irradiation control method for myopia physiotherapy, comprising:

   acquiring an ocular surface image of a user;
   analyzing the ocular surface image to determine state information of a pupil; and
   controlling, based on the state information of the pupil and a red-light irradiation in-eye light power of the user, red-light irradiation on a fundus of the user.

2. The red-light irradiation control method of claim 1, wherein acquiring the ocular surface image of the user includes:
   acquiring the ocular surface image of the user by at least one positioning camera before and/or during red-light irradiation on the fundus of the user.

3. The red-light irradiation control method of claim 1, wherein analyzing the ocular surface image to determine the state information of the pupil includes:
   analyzing the ocular surface image by a neural network model to determine the state information of the pupil.

4. The red-light irradiation control method of claim 1, wherein the state information of the pupil includes a pupil size.

5. The red-light irradiation control method of claim 4, wherein the state information of the pupil further includes a distance of the pupil relative to a red-light irradiation assembly.

6. The red-light irradiation control method of claim 5, wherein the state information of the pupil further includes a position and/or direction of the pupil relative to the red-light irradiation assembly.

7. The red-light irradiation control method of claim 6, further comprising:
   moving the red-light irradiation assembly in at least one moving direction in response to a shift in the position and/or direction of the pupil relative to the red-light irradiation assembly, to provide vertical red-light irradiation on the fundus.

8. The red-light irradiation control method of any one of claims 4 to 7, wherein controlling, based on the state information of the pupil and the red-light irradiation in-eye light power of the user, red-light irradiation on the fundus of the user includes:

   determining a light source power of the red-light irradiation assembly based on the pupil size and the red-light irradiation in-eye light power of the user; and
   controlling the red-light irradiation on the fundus of the user based on the light source power of the red-light irradiation assembly.

9. The red-light irradiation control method of claim 8, wherein the light source of the red-light irradiation assembly includes a non-homogenized light beam, and determining the light source power of the red-light irradiation assembly based on the pupil size and the red-light irradiation in-eye light power of the user includes:
   determining the light source power based on the pupil size, the red-light irradiation in-eye light power of the user, and a power-annulus radius correspondence relationship of the non-homogenized light beam.

10. The red-light irradiation control method of claim 8, wherein the light source of the red-light irradiation assembly includes a homogenized light beam, and determining the light source power of the red-light irradiation assembly based on the pupil size and the red-light irradiation in-eye light power of the user includes:
    determining the light source power based on the pupil size, the red-light irradiation in-eye light power of the user, and a power-annulus radius correspondence relationship of the homogenized light beam.

11. The red-light irradiation control method of claim 8, wherein controlling, based on the state information of the pupil and the red-light irradiation in-eye light power of the user, red-light irradiation on the fundus of the user further includes:
    determining the light source power of the red-light irradiation assembly based on the pupil size, the distance of the pupil relative to the red-light irradiation assembly, and the red-light irradiation in-eye light power of the user.

12. The red-light irradiation control method of claim 8, wherein the light source power of the red-light irradiation assembly ranges from 0.1mw to 1.7mw.

13. The red-light irradiation control method of claim 12, wherein a spectrum of the light source is narrow-band red-light or infrared light, with a center wavelength within the range of 630nm to 850nm, and a width (full width at half maximum) less than 20nm.

14. A red-light irradiation control device for myopia physiotherapy, comprising:

    a processor; and
    a memory having program instructions for controlling red-light irradiation on a fundus stored thereon which, when executed by a processor, cause the method of any one of claims 1 to 13 to be implemented.

15. An apparatus for myopia physiotherapy, comprising:

    a positioning camera configured to capture an ocular surface of a user to generate an ocular surface image;
    a red-light irradiation assembly configured to irradiate red-light on a fundus of the user to implement myopia physiotherapy; and
    the device of claim 14, which is connected to the positioning camera and the red-light irradiation assembly, respectively, and configured to control the red-light irradiating the fundus.

16. A computer-readable storage medium having program instructions for controlling red-light irradiation for myopia physiotherapy stored thereon which, when executed by a processor, cause the method of any one of claims 1 to 13 to be implemented.

100

| Acquire an ocular surface image of a user | S102 |

↓

| Analyze the ocular surface image to determine state information of a pupil | S104 |

↓

| Control, based on the state information of the pupil and a red-light irradiation in-eye light power of the user, red-light irradiation on a fundus of the user | S106 |

**Fig. 1**

200

| Capture an eyeball of a myope by at least one positioning camera to obtain an ocular surface image of the eyeball | S202 |

↓

| Analyze the ocular surface image by a neural network model to obtain a pupil size | S204 |

↓

| Determine the light source power of the red-light irradiation assembly based on the pupil size, the distance of the pupil relative to the red-light irradiation assembly, and the red-light irradiation in-eye light power of the user | S206 |

↓

| Control the red-light irradiation on the fundus of the user based on the light source power of the red-light irradiation assembly | S208 |

**Fig. 2**

300

Capture an eyeball by a plurality of positioning cameras to obtain an ocular surface image of the eyeball — S302

Analyze the ocular surface image by a neural network model to obtain position information of a pupil — S304

Determine a pupil size based on the position information of the pupil — S306

S308

Non-homogenized light beam or homogenized light beam?

Non-homogenized light beam

Homogenized light beam

Determine the light source power based on the pupil size, the red-light irradiation in-eye light power of the user, and a power-annulus radius correspondence relationship of the non-homogenized light beam — S310

Determine the light source power based on the pupil size, the red-light irradiation in-eye light power of the user, and a power-annulus radius correspondence relationship of the homogenized light beam — S312

Control the red-light irradiation on the fundus of the user based on the determined light source power — S314

**Fig. 3**

Memory
402

Processor
403

Control result

**Fig. 4**

400

Positioning camera
501

Red-light irradiation
assembly
502

Memory
402

Processor
403

400

500

**Fig. 5**

## INTERNATIONAL SEARCH REPORT

| International application No. |
|---|
| **PCT/CN2023/107768** |

**A. CLASSIFICATION OF SUBJECT MATTER**

A61N5/06(2006.01)i; A61F9/00(2006.01)i

According to International Patent Classification (IPC) or to both national classification and IPC

**B. FIELDS SEARCHED**

Minimum documentation searched (classification system followed by classification symbols)

IPC: A61N; A61F

Documentation searched other than minimum documentation to the extent that such documents are included in the fields searched

Electronic data base consulted during the international search (name of data base and, where practicable, search terms used)

CNTXT; CNKI; ENTXT; DWPI; 近视, 红光, 图像, 瞳孔, 尺寸, 大小, 功率, 距离, 控制, 调节; myopia, eye, optical, light, pupil, imag+, power, distance, control+, adjust+

**C. DOCUMENTS CONSIDERED TO BE RELEVANT**

| Category* | Citation of document, with indication, where appropriate, of the relevant passages | Relevant to claim No. |
|---|---|---|
| PX | CN 114887233 A (BEIJING AIRDOC TECHNOLOGY CO., LTD.) 12 August 2022 (2022-08-12) description, paragraphs 25-58 | 14-16 |
| PX | CN 114887232 A (BEIJING AIRDOC TECHNOLOGY CO., LTD.) 12 August 2022 (2022-08-12) description, paragraphs 27-67 | 14-16 |
| PX | CN 115245311 A (BEIJING AIRDOC TECHNOLOGY CO., LTD.) 28 October 2022 (2022-10-28) description, paragraphs 34-69 | 14-16 |
| X | CN 114272519 A (AISHIYA HEALTH TECHNOLOGY (SUZHOU) CO., LTD.) 05 April 2022 (2022-04-05) description, paragraphs 48-63, and figures 1-11 | 14-16 |
| Y | CN 114272519 A (AISHIYA HEALTH TECHNOLOGY (SUZHOU) CO., LTD.) 05 April 2022 (2022-04-05) description, paragraphs 48-63, and figures 1-11 | 14-16 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **24 October 2023** | **27 October 2023** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/107768** |

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| Y | CN 112220447 A (SHANGHAI YINGTONG MEDICAL TECHNOLOGY CO., LTD.) 15 January 2021 (2021-01-15)<br>  description, paragraphs 38-63 | 14-16 |
| X | CN 114209990 A (AISHIYA HEALTH TECHNOLOGY (SUZHOU) CO., LTD.) 22 March 2022 (2022-03-22)<br>  description, paragraphs 39-118 | 14-16 |
| A | WO 2021044540 A1 (NEC CORP.) 11 March 2021 (2021-03-11)<br>  entire document | 14-16 |

Form PCT/ISA/210 (second sheet) (July 2022)

## INTERNATIONAL SEARCH REPORT

| International application No. |
| --- |
| **PCT/CN2023/107768** |

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **1-13**
   because they relate to subject matter not required to be searched by this Authority, namely:

   Methods for treatment of the human or animal body (PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2023/107768**

| Patent document cited in search report | | | Publication date (day/month/year) | Patent family member(s) | Publication date (day/month/year) |
|---|---|---|---|---|---|
| CN | 114887233 | A | 12 August 2022 | None | |
| CN | 114887232 | A | 12 August 2022 | None | |
| CN | 115245311 | A | 28 October 2022 | None | |
| CN | 114272519 | A | 05 April 2022 | None | |
| CN | 112220447 | A | 15 January 2021 | None | |
| CN | 114209990 | A | 22 March 2022 | None | |
| WO | 2021044540 | A1 | 11 March 2021 | None | |

Form PCT/ISA/210 (patent family annex) (July 2022)

**EP 4 556 062 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210832712